# FASCICULE DE BREVET EUROPEEN

(11) **EP 0 725 631 B1**
(45) Date de publication et mention de la délivrance du brevet: **02.04.2003**
(21) Numéro de dépôt: 95925887.2
(22) Date de dépôt: 13.07.1995
(51) Int. Cl.: A61K 9/20

(54) **NOUVEAU PROCEDE DE REALISATION DE FORMES PHARMACEUTIQUES SECHES A DELITEMENT QUASIMENT INSTANTANE ET LES FORMES PHARMACEUTIQUES AINSI REALISEES**
NEUES VERFAHREN ZUR HERSTELLUNG VON TROCKENEN PHARMAZEUTISCHEN FORMEN MIT SCHNELLAUFLOESBARKEIT UND SO HERGESTELLTE PHARMAZEUTISCHE FORMEN
NOVEL METHOD FOR PREPARING DRY PHARMACEUTICALS CAPABLE OF VIRTUALLY INSTANTANEOUS DISINTEGRATION, AND RESULTING PHARMACEUTICALS

(30) Priorité: 15.07.1994 FR 9408811
(43) Date de publication de la demande: 14.08.1996
(73) Titulaire: Vacher, Dominique, 06600 Antibes (FR)
(72) Inventeur: Vacher, Dominique, 06600 Antibes (FR)
(74) Mandataire: Burtin, Jean-François
(86) Numéro de dépôt international: FR9500947
(87) Numéro de publication internationale: WO96002237

(56) Documents cités:
- EP-A- 0 273 005

## Description

La présente invention se rapporte au domaine de la chimie thérapeutique et notamment au domaine de la pharmacie galénique.

Elle a plus particulièrement pour objet un procédé de réalisation de formes galéniques sèches notamment comprimées, susceptibles de se dissoudre dans un laps de temps très court, sinon instantanément.

Elle a spécifiquement pour objet un procédé de réalisation de formes pharmaceutiques solides résultant d'une compression de poudres, et en particulier, de comprimés, de tablettes, de pastilles ou de dragées, dans lequel le ou les principes actifs, préalablement enrobés dans un agent liant, sont mélangés à un agent gonflant du type cellulosique et à un ou plusieurs agents diluants a hydrosolubles, de sorte que la forme pharmaceutique se délite au contact de l'eau ou des liquides biologiques comme la salive, d'une manière pratiquement instantanée.

Un comprimé est une forme galénique administrable par voie orale soit en vue d'un traitement local de la sphère bucco-pharyngée, soit en vue d'un traitement systémique après absorption des substances pharmacologiquement actives.

Si avaler des comprimés, bien que quelque fois peu aisé lorsque les comprimés sont gros, ou mal commode, lorsque leur forme s'y prête mal, ne pose pas, en principe, de problème majeur chez l'adulte en bonne santé, il n'en va pas de même pour le nourrisson et l'enfant.

En effet, il est toujours délicat d'administrer un médicament par voie orale chez un nourrisson et d'avoir la connaissance précise de la dose effectivement ingérée. Il arrive que l'enfant recrache une partie du produit lorsque celui-ci est administré directement ou qu'il n'absorbe pas l'intégralité de la quantité de substance, mise dans le biberon. L'enfant reçoit donc un traitement sous-dosé.

A l'inverse, la mère peut être amenée à augmenter la fréquence d'administration pour compenser les pertes de produit, et l'enfant peut alors recevoir un surdosage.

Dans tous les cas, ces difficultés d'administration entraînent un ralentissement, voire même une suppression du traitement, ce qui n'est pas toujours souhaitable, comme c'est le cas par exemple pour un traitement par les antibiotiques qui doit s'effectuer pendant un délai minimum.

Ces conditions d'administration, difficiles chez le nourrisson, peuvent être retrouvées dans certains cas chez l'adulte où la déglutition est rendue difficile soit du fait de l'âge du sujet, soit du fait de son état de santé (traumatisme facial, cancer de la sphère bucco-pharyngée, mauvais état de la dentition ...).

Un des moyens connus pour tenter de résoudre ces problèmes de déglutition consiste à déliter et à dissoudre, préalablement, le comprimé dans de l'eau, puis à ingérer le médicament sous sa forme dissoute.

Le document EP-A-0 271 005 se rapporte à des tablettes dispersibles pour former dans l'eau une suspension homogène à haute viscosité. Ces tablettes de poids élevé sont obtenus essentiellement en mélangeant des microparticules contenant au moins un principe actif, au moins un agent désintégrant sous forme de granulés secs et un agent gonflant capable de générer une viscosité importante au contact de l'eau sous forme de granulés obtenus par voie humide.

C'est ainsi qu'il existe actuellement des comprimés effervescents dans lesquels l'excipient comprend de l'acide citrique et du bicarbonate de sodium qui réagissent ensemble, en présence d'eau pour produire du gaz carbonique et provoquent, ainsi le délitement du comprimé et la dissolution, plus ou moins complète, du principe actif et de l'excipient.

La solution aqueuse gazeuse ainsi obtenue a un goût très inhabituel pour l'enfant, soit acide, soit franchement alcalin, ce qui conduit celui-ci à en refuser l'ingestion. En outre, le temps de délitement du comprimé est relativement long (environ 2 mn).

Il faut signaler également que certains principes actifs ne se prêtent pas à une dissolution préalable extra corporelle.

On connaît, par ailleurs, des comprimés à délitement rapide qui, pour parvenir à ce but, contiennent proportionnellement de grandes quantités d'excipient par rapport aux quantités de principe actif présent.

Ceci a pour conséquence directe que de tels comprimés ont des tailles importantes, ce qui ne facilite pas leur administration, sous forme solide, par voie orale.

L'invention définie dans le brevet français antérieur 2.638.971, au nom du Demandeur visait à remédier à ces inconvénients en fournissant un comprimé à délitement et/ou dissolution rapide du type constitué par au moins un principe actif et par un excipient qui soit de petite taille et qui soit administrable par voie orale sous forme solide et de façon précisément dosée, à des sujets dont la déglutition est difficile et, notamment, à des nourrissons.

A cet effet, le comprimé à délitement et dissolution quasi-instantané qu'elle concernait était caractérisé par le fait que l'excipient comprenait essentiellement un composé macromoléculaire polyosidique réticulé et un agent gonflant.

Après de nombreux essais, le Demandeur avait ainsi constaté, de manière surprenante, qu'il existait une synergie d'activité entre le composé macromoléculaire polyosidique et l'agent gonflant dans les phénomènes de délitement et de dissolution.

Le comprimé ainsi obtenu possédait la propriété de se déliter et de se dissoudre en un temps extrêmement court d'environ 30 secondes, et ceci à l'intérieur même de la cavité buccale, en utilisant exclusivement le liquide présent localement pour réaliser ce changement d'état.

Il permettait selon ce brevet, d'administrer facilement aux nourrissons, la quantité exacte de médicament prescrite.

Néanmoins, les exemples fournis dans ce brevet, à titre d'illustration de l'invention, montraient que ce procédé ne pouvait être appliqué qu'à la réalisation de comprimés de très petite taille et ne renfermant qu'une quantité faible de principe actif. C'est ainsi que des comprimés renfermant 50 mg d'aspirine comme décrit dans ce brevet, ne peuvent être destinés qu'à des sujets extrêmement jeunes puisque la posologie usuelle journalière d'aspirine est de 25 à 50 mg/kg sans dépasser 80 mg/kg pour des enfants de 0 à 30 mois.

Une telle formulation laisserait donc supposer une administration d'un nombre considérable de comprimés.

La présente invention vise à améliorer la solution du problème technique défini dans le brevet français 2.638.971 mais imparfaitement résolu, en permettant une relative généralisation du principe actif dont l'administration est envisagée quelque soit la saveur ou le degré de solubilité du principe actif et en n'étant pas lié par le problème de la taille et du poids du comprimé ainsi réalisé, en raison de son très facile délitement au contact de la salive.

Les essais effectués par le demandeur ont montré que des comprimés à délitement quasi-instantané pouvaient être réalisés par une technique différente de celle définie dans le brevet 2.638.971. Pour ce faire, le principe actif est au préalable enrobé dans un excipient hydrodispersible et notamment par un agent liant du type alcoylcellulose, de façon à ce que chaque grain ou chaque cristal, après compression, soit aisément mouillé par les liquides aqueux comme par exemple, la salive. Ensuite, le principe actif enrobé est dispersé dans un diluant formé principalement d'un carboxyméthyl cellulose réticulée comme celle désignée sous la dénomination Croscarmellose sodique où l'agent réticulant est l'acide monochloracétique, qui possède un fort pouvoir de gonflement, d'un polyol très soluble dans l'eau et d'un ou plusieurs agents diluants.

Dans cette matrice, l'agent diluant, de préférence la cellulose micro cristalline, remplit une autre fonction que celle définie dans le brevet français 2.638.971 antérieur. Elle n'agit pas comme agent de gonflement, ce qui parait, de toute façon, inhabituel et même peut être douteux en raison de son insolubilité dans l'eau, mais simplement comme agent d'éclatement des comprimés.

Le polyol est choisi parmi les glucitols et les diglucitols comme le sorbitol, le mannitol, le xylitol ou le lactitol.

On réalise ainsi une matrice de forme sèche, de désagrégation très rapide et où le polyol joue, en plus, un rôle d'agent de sapidité en dissimulant ou en enrobant la saveur propre du principe actif. Ainsi, la quantité de principe actif et sa saveur propre, ne constituent plus un obstacle à l'administration et à la déglutition du médicament.

Parmi les principes actifs qu'il est possible d'incorporer aux formes pharmaceutiques sèches, selon l'invention, on pourra citer des médicaments antalgiques simples ou mélangés comme l'aspirine, le paracétamol, l'ibuprofène, la codéine, le dextropropoxyphène, le benorilate, le glucuronamide, la phénacétine, l'éthobenzamide, la floctafénine, l'emorfazone, la noramidopyrine ainsi que les mélanges de deux ou plusieurs principes actifs comme le dextropropoxyphène+paracétamol, l'aspirine+paracétamol, l'aspirine+paracétamol+ codéine, l'ibuprofène+codéine, l'aspirine+caféine, l'aspirine+éphédrine, le glucuronamide+aspirine, le paracétamol+prométhazine ou l'aspirine+acétéamine.

On pourra également incorporer :
- des agents antispasmodiques comme l'atropine et son N-oxyde, la dihexyvérine, le bromure de N-butylhyoscine, le méthylsulfate de Tiemonium, le chlordiazépoxyde, le chlordiazepoxyde+bromure de clinidium, le bromure de pinaverium, l'oxyphencyclimine, le phloroglucinol, la camilophyline, la difémérine, la camilophyline+métamizole (sel de sodium),
- des agents anti-ischémiques comme la dihydroergotoxine, la dihydroergocryptine, la nicergoline, la trimétazidine, l'éburnamonine ou le naftidrofuryl,
- des agents décongestionnants comme les associations pseudoéphédrine+ paracétamol, buzépide+cloanizine+noréphédrine, Triprolidine+ pseudoéphédrine +paracétamol,
- des agents nootropiques comme le Piracetam, l'Amiracetam ou l'Oxyracétam,
- des agents vasodilatateurs et/ou antihypertenseurs comme la Nicardipine, la Nifédipine, la Nimodipine, la Nisoldipine et des produtis analogues,
- des agents antihistaminiques comme la terfénadine, la loratidine ou la cétirizine
- des agents antibiotiques ou antibactériens comme la céfazoline, la tyrocidine, la gramicidine, la cefaloglycine, l'amipicilline, la sulfamicilline, l'amoxicilline ou les associations avec un inhibiteur de β-lactamasee, les sulfamides comme le sulfamétoxazole, les agents antibactériens comme la péfloxacine, la norfloxacine ou l'ofloxacine.
- des agents anti-migraineux comme la dihydroergotamine
- des agents anti-vertigineux comme la betahistine et ses sels
- des agents myorelaxants comme la chlormezanone

L'exemple suivant illustre l'invention sans toutefois la limiter à cette réalisation particulière :

### EXEMPLE I

### Comprimé à dissolution instantanée à 500 mg de paracétamol.

### Composition :

| | |
|---|---|
| Paracétamol enrobé par de l'éthyl cellulose correspondant à 500 g | |
| de paracétamol pur | 540,5 g |
| Aspartame | 15 g |
| Croscarmellose | 90 g |
| Arôme orange | 20 g |
| Acide citrique | 30 g |
| Xylitol | 100 g |
| Cellulose microcristalline | 99,5 g |
| Stéarate de magnésium | 5 g |
| pour 1000 comprimés | |

### EXEMPLE II

### Comprimés à dissolution instantanée à base de Triprolidine et de pseudoéphédrine

| | |
|---|---|
| Chlorhydrate de Triprolidine | 2,5 g |
| Chlorhydrate de Pseudoéphédrine Paracétamol enrobé par de l'éthylcellulose | 50 g |
| correspondant à 300 g de paracétamol | 325 g |
| Croscarmellose sodique | 105 g |
| Arôme orange sur silice | 20 g |
| Acide citrique | 25 g |
| Mannitol | 125g |
| Cellulose microcristalline commercialisée sous la marque AVICEL X100 | 110 g |
| Talc | 4 g |
| pour 1000 comprimés | |

## Revendications

1. Procédé de réalisation de formes pharmaceutiques solides et permettant le délitement instantané dans la cavité buccale, dans lequel le ou les principes actifs préalablement enrobés dans une alcoylcellulose comme agent liant hydrodispersible, sont mélangés à un agent à fort pouvoir de gonflement du type carboxyméthylcellulose, et à un polyol soluble dans l'eau et à un ou plusieurs agents diluants et ensuite sont comprimés pour former des comprimés à délitement instantané.

2. Procédé selon la revendication 1° **caractérisé en ce que** l'alcoylcellulose est l'éthylcellulose.

3. Procédé selon la revendication 1° **caractérisé en ce que** la carboxyméthylcellulose est une carboxyméthylcellulose réticulée.

4. Procédé selon la revendication 1° **caractérisé en ce que** le polyol soluble dans l'eau est choisi parmi les glucitols et les diglucitols.

5. Procédé selon la revendication 1° ou la revendication 4° **caractérisé en ce que** le polyol est choisi dans le groupe constitué par le xylitol, le mannitol, le sorbitol et le lactitol.

6. Procédé selon la revendication 1° **caractérisé en ce que** l'agent diluant est la cellulose microcristalline.

7. Procédé selon l'une quelconque des revendications 1 à 6 **caractérisé en ce que** le principe actif contenu dans la forme pharmaceutique solide est choisi dans le groupe constitué par les antalgiques, les agents antispasmodiques, les agents anti-ischémiques, les agents décongestionnants, les agents nootropiques, les agents vasodilatateurs et/ou antihypertenseurs, les agents antibiotiques ou antibactériens, les agents antimigraineux, les agents antivertigineux et les agents myorelaxants.

8. Procédé selon la revendication 7° **caractérisé en ce que** l'antalgique est choisi dans le groupe formé de l'aspirine, du paracétamol, de l'ibuprofène, de la codéine, du dextropropoxyphène, du benorilate, du glucuronamide, de la floctafénine, de l'émorfazone, de la nor-amidopyrine ainsi que de leurs mélanges.

## Claims

1. A process for achieving solid pharmaceutical forms and allowing the instantaneous desintegration in the oral cavity, wherein this or the active ingredients previously coated in an alkylcellulose as a hydrodispersible binding agent, are admixed to an agent endowed with a strong swelling power of the type of carboxymethylcellulose, to a water soluble polyol and to one or several diluents and they are compressed to form tablets with instantaneous disintegration.

2. A process according to claim 1, **characterized in that** the alkylcellulose is ethylcellulose.

3. A process according to claim 1, **characterized in that** the carboxymethycellulose is a cross linked carboxymethylcellulose

4. A process according to claim 1, **characterized in that** the water-soluble polyol is selected among glucitols and diglucitols.

5. A process according to claim 1 or claim 4 **characterized in that** the polyol is selected from the group constisting of xylitol, mannitol, sorbitol and lactitol.

6. A process according to claim 1 **characterized in that** the diluting agent is microcristalline cellulose.

7. A process according to any of the claims 1 to 6 , **characterized in that** the active ingredient contained in the solid pharmaceutical form, is selected from the group constituted by the analgetics, antispamodic agents, antiischemic agents, decongesting agents, nootropic agents, vasodilating agents and/or antihypertensive agents, antibiotics, or antibacterial agents, anti headache agents, antivetigo agents and myorelaxing agents.

8. Process according to claims 7 **characterized in that** the analgetice is selected from the group formed by paracetamol, ibuprofen, codein, dextropoxyphen, benorilate, glucuronamide, floctafenine, emorfazone, noramidopyrine and the mixtures thereof.

## Patentansprüche

1. Verfahren zur Herstellung eines pharmazeutischen Stoffes, der als Feststoff vorliegt, und der in der Mundhöhle sofort zergeht, bei dem der oder die aktiven Grundbestandteile, zuvor eingehüllt in das in Wasser verteilbare Bindemittel Alkylzellulose, vermischt werden mit einem Mittel, das ein starkes Quellverhalten aufweist, wie Carboxymethylzellulose, und einem wasserlölichen Polyalkohol und einem oder mehreren Verdünnungsmitteln und dann zu Tabletten verpresst werden, die eine sofortige Löslichkeit aufweisen.

2. Verfahren nach Anspruch 1, **dadurch gekennzeichnet, dass** als Alkyzellulose Ethylzellulose verwendet wird.

3. Verfahren nach Anspruch 1, **dadurch gekennzeichnet, dass** als Carboxymethylzellulose eine vemetze Carboxymethylzellulose verwendet wird.

4. Verfahren nach Anspruch 1, **dadurch gekennzeichnet, dass** der wasserlösliche Polyalkohol aus den Glucitolen und den Diglucitolen ausgewählt wird.

5. Verfahren nach Anspruch 1 oder Anspruch 4, **dadurch gekennzeichnet, dass** der Polyalkohol aus der Gruppe, die durch Xylitol, Manitol, Sorbitol und Lactitol gebildet wird, ausgewählt wird.

6. Verfahren nach Anspruch 1n **dadurch gekennzeichnet, dass** als Verdünnungsmittel mikrokristalline Zellulose verwendet wird.

7. Verfahren nach einem der Ansprüche 1 bis 6, **dadurch gekennzeichnet, dass** die im pharmazeutischen Stoff, der als Feststoff vorliegt, enthaltenen aktiven Grundbestandteile aus der Gruppe ausgewählte werden, die sich aus schmerzstillenden Mitteln, krampfstillenden Mitteln, Mitteln gegen Blutleere, Entzündungshemmern, Nootropika, gefäßerweiternden und/oder anspannungsmildernden Mitteln, antibiotischen oder antibakteriellen Mitteln, Mitteln gegen Migräne, schwindelhemmenden Mitteln und muskelenspannenden Mitteln zusammensetzt.

8. Verfahren nach Anspruch 7, **dadurch gekennzeichnet, dass** das schmerzstillende Mittel aus der Gruppe ausgewählt wird, die von Aspirin, Paracetamol, Ibuprofen, Codein, Dextropropoxyphen, Benorilat, Glucuronsäure, Floctafenin, Emorfazone, Noramidopyrin wie auch von Mischungen daraus gebildet wird.
